# EUROPEAN PATENT SPECIFICATION

(11) **EP 0 736 724 B1**
(45) Date of publication and mention of the grant of the patent: **24.03.1999**
(21) Application number: 95203417.1
(22) Date of filing: 08.12.1995
(51) Int. Cl.: F16M 11/24, A61N 5/06

(54) **Stand**
Stativ
Support

(30) Priority: 09.12.1994 NL 9402095
(43) Date of publication of application: 09.10.1996
(73) Proprietor: ALISUN EUROPE B.V., 1704 RX Heerhugowaard (NL)
(72) Inventor: Innikel, Quintijn, ir., NL-1054 XC Amsterdam (NL)
(74) Representative: 't Jong, Bastiaan Jacobus

(56) References cited:
- DE-A- 3 214 194
- GB-A- 2 201 503
- US-A- 4 195 578
- US-A- 4 305 560
- US-A- 4 740 707

## Description

The present invention relates to a stand for height adjustable support of an object, such as a sun ray lamp of a sun bed, comprising:
- a frame to be placed on a horizontal surface; and
- an arm connected with a bottom end to said frame close to a corner of the frame for pivoting round a pivot shaft extending substantially parallel to an adjacent straight first side of the frame and carrying said object on the free end thereof.

A stand of the kind described above is known from the American patent 4,305,460. Said stand comprises an upright frame of independent tubes, each having a bent portion between respective ends thereof, where adjacent tubes in the stand are connected by means of a friction coupling.

As a result thereof the prior art stand is very unstable and susceptable to collapse, when it is used to support a load. This is due to the fact, that a load bigger than the resistance from the friction couplings will result in the tubes rotating relative to each other under influence of such a load, leading to collapse in the direction of the aforementioned horizontal surface.

Also a stand for a sun ray lamp of the kind described above is known from the published German patent application 3,214,194. This stand comprises an upright frame of tubes in a vertical plane, where said arm is connected to a top side thereof. The arm comprises two protrusions, between which the object, the sun ray lamp, is arranged. As a result primarily of the weight of the object to be carried by the arm, this prior art stand is sizable and relatively heavy.

The invention has for its object to provide a stand of the type described above, which has a simple configuration, consists of only a few components, and can therefore be produced at relatively low costs.

This object is achieved in a stand according to the present invention, in that said frame comprises a first one-piece tube bent in a U-shape defining three sides of a substantially rectangular base area, and having a first leg comprising said first substantially straight side, said arm comprises a second one-piece tube bent in an L-shape, where a leg of said second tube comprises a second substantially straight side, said first and second sides being assembled to pivot relative to one another, and arresting means arranged between the end of the leg opposite the first leg and said arm for holding said arm in various positions relative to said first frame.

In a stand according to the present invention the substantially rectangular frame comprises a first tube bent in the substantially rectangular base shape, can be placed on the horizontal surface, in such a way, that only said arm is upright. The arm can pivot up and down relative to and from said frame and therefore to said horizontal surface, and therefore the object carried by the arm formed by the second tube is supported in a height adjustable manner. By pivoting the second tube downward the object supported thereby is lowered to a position very near the horizontal support surface, in which state the stand with the object supported thereby requires very little space. Also resistance of the stand to collapse has been improved. When the object is a sun ray lamp or an other kind of load on the stand, this is a particular advantage.

An additional advantage is, that said stand has an appealing appearance, resulting from the bent shape of the first tube flowing over into the bent shape of the second tube.

Preferably the second tube comprises a third tube end located opposite to said second tube end parallel thereto, where said object is assembled with said third tube and to rotate relative thereto. As a result the object can rotate around an axis substantially parallel to the support surface, on which the frame is placed. Especially for use in a sun bed the sun ray lamp can simply be positioned in this manner, for instance parallel to a person lying on a bed or the like. Hence the sun ray lamp can be positioned parallel to this person, as well as at a suitable distance to this person.

Preferably the arresting means comprise a gas spring. With a suitable choice of the characteristics of the gas spring the object can be supported in a balanced manner, in such a way, that a very easily usable apparatus can be obtained with the stand according to the present invention.

When the frame carries a castor close to each of its corners, as it preferably does, the stand can easily be positioned.

The invention will be further elucidated in the following description with reference to the annexed figures.
Figure 1 shows a sunbed in which an embodiment of a stand according to the invention is applied.

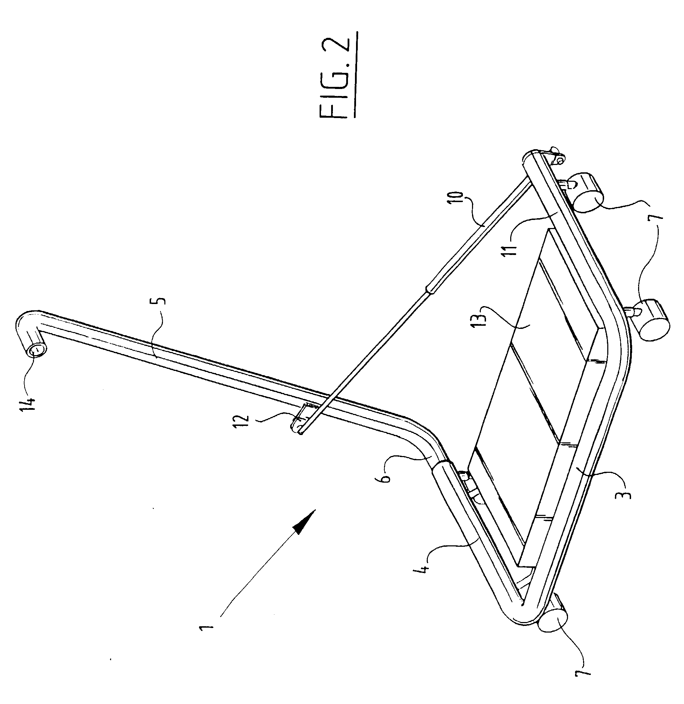

Figure 2 shows the stand of the sunbed of figure 1 in a view according to arrow II in figure 1.

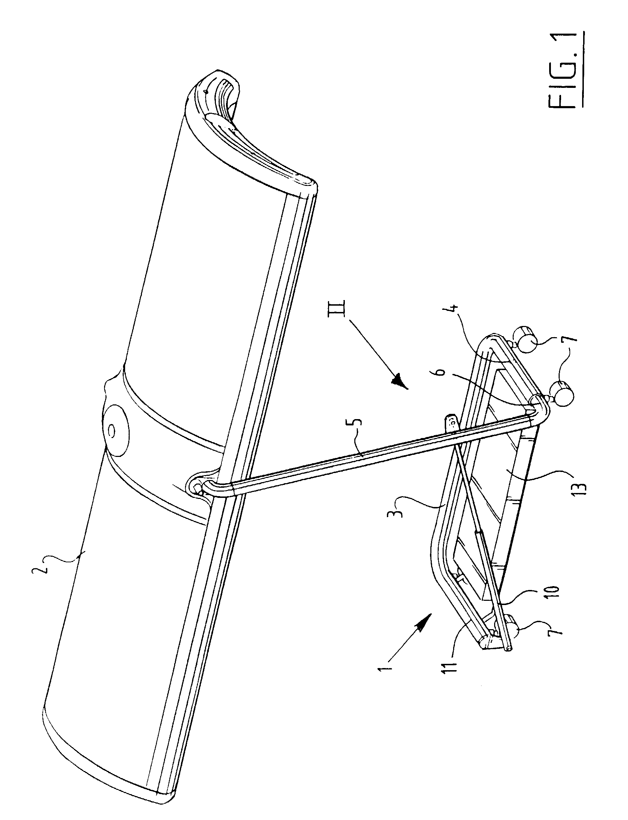

The sunbed of figure 1 comprises a stand 1 and a sun ray lamp 2 supported thereby, in which a plurality of UV tube lights is fitted in a manner not further shown here. The tube lights radiate light on the concave side of the sun ray lamp 2.

During use the lamp 2 is positioned such that the concave surface is situated close to the body of the user, in order to radiate UV light thereon.

The stand 1 according to the shown embodiment of the invention comprises a first, U-shaped bent tube 3 and a second tube 5 with an L-shaped part. A protruding leg 6 of the L-shaped part of the second tube 5 is placed into a leg 4 of the U-tube 3. The diameter of the tube part 6 is chosen herein such that it fits closely but still movably in the leg 4 of the U-tube 3.

The U-tube 3 is provided on four corners with wheels 7, which in this example are castors.

Arranged in this embodiment between the tube 5 and the end of the leg 11 lying opposite the leg 4 of the U-tube is a gas spring 10 which engages on a support 12 of the L-tube 5. By means of this gas spring 10 the tube 5 is urged into an erect position. The gas spring 10 has a spring characteristic such that a balancing of the sun ray lamp 2 substantially occurs so that it can be very easily adjusted to any desired height without clamping connections having to be released and refastened.

The placing of the leg 6 of the L-tube in the leg 4 of the U-tube can optionally be such that some resistance to rotation occurs. Some force must then be applied to change the height of the sun ray lamp 2.

A weight element 13 is arranged in the U-tube 3 so that the sunbed shown in figure 1 becomes a stable unit.

The end of the L-tube 5 is bent so that another end portion 14 is formed which extends parallel to the leg 6 of the tube 5. The sun ray lamp 2 is mounted rotatably on this portion 14.

The invention is therefore not limited to the embodiment shown in the figures.

## Claims

1. Stand (1) for height adjustable support of an object (2), such as a sun ray lamp of a sun bed, comprising:
- a frame (3) to be placed on a horizontal surface; and
- an arm (5) connected with with a bottom end to said frame close to a corner of the frame for pivoting round a pivot shaft extending substantially parallel to an adjacent straight first side (4) of the frame and carrying said object on the free end thereof;
**characterized in that**
- said frame (3) comprises a first one-piece tube bent in a U-shape defining three sides of a substantially rectangular base area, and having a first leg comprising said first substantially straight side (4),
- said arm (5) comprises a second one-piece tube bent in an L-shape, where a leg of said second tube (5) comprises a second substantially straight side (6), said first (4) and second (6) sides being assembled to pivot relative to one another, and
- arresting means (10) arranged between the end of the leg opposite the first leg and said arm (5) for holding said arm (5) in various positions relative to said first frame (3).

2. Stand as claimed in claim 1, **characterized in that** said second tube (5) comprises a third tube end (14) located opposite to said second tube end (6) and parallel thereto, where said object (2) is assembled with said third tube end (14) to rotate relative thereto.

3. Stand as claimed in claim 1 or 2,
**characterized in that** said arresting means comprise a gas spring (10).

4. Stand as claimed in claim 1, 2 or 3,
**characterized in that** said frame (3) carries a castor (7) close to each of its corners.

## Patentansprüche

1. Stativ (1) für höheneinstellbare Halterung eines Objektes (2), wie beispielsweise einer großen UV-Lampe einer Sonnenbank, mit:
- einem Rahmen (3), der auf einer horizontalen Oberfläche zu plazieren ist, und
- einem Arm (5), der an seinem freien Ende das Objekt trägt und der mit einem unteren Ende des Rahmens nahe der Ecke des Rahmens verbunden ist, um um eine Schwenkachse zu schwenken, die sich im wesentlichen parallel zu einer benachbarten geraden ersten Seite (4) des Rahmens erstreckt, dadurch **gekennzeichnet,** daß
- der Rahmen (3) ein erstes einstückiges Rohr aufweist, das in U-Form gebogen ist, drei Seiten einer im wesentlichen rechteckigen Grundfläche definiert und einen ersten Schenkel hat, der die im wesentlichen gerade erste Seite (4) aufweist;
- der Arm (5) ein zweites einstückiges Rohr aufweist, das in L-Form gebogen ist, wobei ein Schenkel des zweiten Rohres (5) eine zweite im wesentlichen gerade Seite (6) aufweist, wobei die erste (4) und die zweite (6) Seite so zusammengebaut sind, daß sie relativ zueinander schwenken, und
- Haltemittel (10) zwischen dem Ende des Schenkels gegenüber dem ersten Schenkel und dem Arm (5) angeordnet sind, um den Arm (5) in verschiedenen Positionen relativ zum ersten Rahmen (3) zu halten.

2. Stativ nach Anspruch 1, dadurch **gekennzeichnet,** daß das zweite Rohr (5) ein drittes Rohrende (14) aufweist, das gegenüber dem zweiten Rohrende (6) und parallel zu diesem liegt, wobei das Objekt (2) an dem dritten Rohrende (14) relativ zu diesem drehbar montiert ist.

3. Stativ nach Anspruch 1 oder 2, dadurch **gekennzeichnet,** daß die Haltemittel eine Gasfeder (10) aufweisen.

4. Stativ nach Anspruch 1, 2 oder 3, dadurch **gekennzeichnet,** daß der Rahmen (3) in der Nähe jeder seiner Ecken eine schwenkbare Laufrolle (7) trägt.

## Revendications

1. Pied (1) pour un support ajustable en hauteur d'un objet (2) tel qu'une lampe à bronzer d'un lit de bronzage, comprenant :
- un châssis (3) destiné à être placé sur une surface horizontale ; et
- un bras (5) relié à une extrémité inférieure dudit châssis à proximité d'un coin du châssis pour pivoter autour d'une tige de pivot s'étendant sensiblement parallèlement à un premier côté droit adjacent (4) du châssis et portant ledit objet sur son extrémité libre ;
caractérisé en ce que
- ledit châssis (3) comprend un premier tube formé d'une pièce, plié en forme de U définissant trois côtés d'une région de base sensiblement rectangulaire, et ayant une première jambe comprenant ledit premier côté sensiblement droit (4),
- ledit bras (5) comprend un second tube formé d'une pièce, plié en forme de L où une jambe dudit second tube (5) comprend un second côté sensiblement droit (6), lesdits premier (4) et second (6) côtés étant assemblés pour pivoter l'un par rapport à l'autre, et
- des moyens d'arrêt (10) agencés entre l'extrémité de la jambe opposée à la première jambe et ledit bras (5) pour tenir ledit bras (5) dans différentes positions par rapport audit premier châssis (3).

2. Pied selon la revendication 1, caractérisé en ce que ledit second tube (5) comprend une troisième extrémité de tube (14) située à l'opposé de ladite seconde extrémité de tube (6) et parallèle à celle-ci où ledit objet (2) est assemblé avec la troisième extrémité de tube (14) de manière à tourner par rapport à celle-ci.

3. Pied selon la revendication 1 ou 2, caractérisé en ce que lesdits moyens d'arrêt comprennent un ressort à gaz (10).

4. Pied selon la revendication 1, 2 ou 3, caractérisé en ce que ledit châssis (3) porte une roulette (7) proche de chacun de ses coins.
